# EUROPEAN PATENT SPECIFICATION

(11) **EP 1 536 837 B1**
(45) Date of publication and mention of the grant of the patent: **15.09.2010**
(21) Application number: 03763548.9
(22) Date of filing: 16.07.2003
(51) Int. Cl.: A61K 47/36, A61K 47/38, A61L 27/20, A61L 27/38, A61P 19/02

(54) **COMPOSITION FOR CYTOCOMPATIBLE, INJECTABLE, SELF-GELLING CHITOSAN SOLUTIONS FOR ENCAPSULATING AND DELIVERING LIVE CELLS OR BIOLOGICALLY ACTIVE FACTORS**
ZUSAMMENSETZUNG FÜR DIE HERSTELLUNG ZELLKOMPATIBLER, INJIZIERBARER, SELBSTGELIERENDER CHITOSAN LÖSUNGEN ZUM EINKAPSELN UND VERABREICHEN VON LEBENDEN ZELLEN ODERBIOLOGISCH AKTIVEN FAKTOREN
COMPOSITION POUR SOLUTIONS DE CHITOSANE CYTOCOMPATIBLES, INJECTABLES ET AUTO-GELIFIANTES DESTINEES A L'ENCAPSULATION ET A L'ADMINISTRATION DE CELLULES VIVANTES OU DE FACTEURS BIOLOGIQUEMENT ACTIFS

(30) Priority: 16.07.2002 US 395991 P
(43) Date of publication of application: 08.06.2005
(73) Proprietor: Bio Syntech Canada Inc., Laval, Québec H7V 4B3 (CA)
(72) Inventor: HOEMANN, Caroline, Montréal, Québec H4B 2H4 (CA); CHENITE, Abdellatif, Kirkland, Québec H9H 4H6 (CA); BUSCHMANN, Michael, Montréal, Québec H4B 2H4 (CA); SERRIQI, Alessio, Montréal, Québec H2V 3G7 (CA); SUN, Jun, Montréal, Québec H1T 1S5 (CA)
(74) Representative: Ahner, Francis
(86) International application number: PCT/CA2003/001069
(87) International publication number: WO 2004/006961

(56) References cited:
- WO-A-99/47186
- WO-A-03/042250
- US-A- 4 895 724
- US-A- 5 489 401
- FRANCIS SUH J-K ET AL: "Application of chitosan-based polysaccharide biomaterials in cartilage tissue engineering: a review" BIOMATERIALS, ELSEVIER SCIENCE PUBLISHERS BV., BARKING, GB, vol. 21, no. 24, 15 December 2000 (2000-12-15), pages 2589-2598, XP004217422 ISSN: 0142-9612
- SUTO SHINICHI ET AL: "Chemical crosslinking of hydroxypropyl cellulose and chitosan blends" J APPL POLYM SCI;JOURNAL OF APPLIED POLYMER SCIENCE SEP 26 1996 JOHN WILEY & SONS INC, NEW YORK, NY, USA, vol. 61, no. 13, 26 September 1996 (1996-09-26), pages 2273-2278, XP002258373
- RUEL-GARIEPY E ET AL: "CHARACTERIZATION OF THERMOSENSITIVE CHITOSAN GELS FOR THE SUSTAINEDDELIVERY OF DRUGS" INTERNATIONAL JOURNAL OF PHARMACEUTICS, AMSTERDAM, NL, vol. 203, 10 August 2000 (2000-08-10), pages 89-98, XP000997714 ISSN: 0378-5173

## Description

### BACKGROUND OF THE INVENTION

The invention relates to a composition and method of application to encapsulate live cells with a neutral isotonic chitosan gel solution that is able to solidify in situ with the aid of a cytocompatible cross-linker consisting of glyoxal-treated hydroxyethyl cellulose dissolved in a physiological medium to aid tissue regeneration or wound-healing.

### Description of Prior Art

### 1) Chitosan liquid solutions

Chitosan with a degree of deacetylation (DDA) between 50%DDA and 100%DDA can be completely solubilized in acidic aqueous solutions having a pH below the apparent chitosan pKa (pH 2.5 to pH 6.0). Such chitosan solutions are incompatible with cell viability. Attempts to raise the pH to cytocompatible levels with most buffers will cause the solution to precipitate, unless as shown previously by the Applicant, the buffer used is a polyol-phosphate (glycerol phosphate, GP) dibasic salt (Chenite Patent publication WO 99/07416). Chitosan/GP liquid solutions of pH 6.8 to 7.2 are cytocompatible and thermogelling. However, Chitosan/GP solutions capable of gelling at temperatures near body-temperature contain salt concentrations well beyond cytocompatible limits (8% disodium-GP is - 360 mM, or 1080 mOsm). The thermogelling temperature is inversely proportional to the GP concentration, such that lowering the GP concentration to isotonic levels of salt (3% disodium-GP, -126 mM, 378 mOsm) results in a solution that is thermogelling at non-physiological temperatures, above 65°C. Therefore, cytocompatible liquid chitosan solutions may be generated using acid-solubilized chitosan brought to cytocompatible pH and tonicity with GP, however these solutions are unable to gel in an open body cavity or petri.

### 2) Cross-linked gels from liquid chitosan solutions.

Many chemical cross-linkers have been proposed to form solid gels from liquid chitosan, including glyoxal (Freeman USP5,489,401, 1996), glutaraldehyde (Hsien T-Y and Rorrer GL Ind Eng Chem Res. 36: 3631-3638, 1997; Oyrton AC Montiero Jr and Airoldi C. Internat. J. Biological Macromolecules. 26:119-128, 1999; Kumbar, SG, et al., J. Microencapsulation 19: 173-180, 2002; and Mi, F-L, et al., Biomaterials. 23:181-191, 2002), squarate (DeAngelis AA, et al., Macromolecules 31:1595-1601, 1998), oligo(ethylene oxide) (Rogovina SZ, et al., Polymer Science, 43: 265-268, 2001), tetramethoxy propane (Capitani D, et al., Carbohydrate Polymers 45:245-252, 2001), and genepin (Mi, F-L, et al., Biomaterials. 23:181-191, 2002). A prior invention has also taught that neutral chitosan solutions may be induced to gel using glyoxal solutions between 0.01% and 10% by weight glyoxal or other bifunctional cross-linker (Chenite et al. WO02/40070). However, these concentrations of glyoxal are toxic to cells.

A method for entrapping live cells in a chitosan gel using pH-dependent precipitation and non-covalent cross-links, in contrast to a chemical cross-linker, has been previously invented (Aebischer et al., US Patent 5,871,985). However this pH-dependent gellation mechanism leads to a form of chitosan paste that lacks the adhesive and mechanical properties of the chitosan gels described herein, and would have limited use in the domain of cartilage repair applications where the gel is to be applied to a tissue surface in an open body cavity such as the synovial joint.

In a previous invention (WO02/00272) a cytocompatible chitosan-GP liquid solution was proposed for use in cell encapsulation for tissue repair or regeneration based on thermogelling properties of the liquid chitosan-GP solution. Retention of viable cells in a solid chitosan gel with a composition of chitosan-GP, glucosamine, and hydroxyethyl cellulose was described. In a separate publication (Li and Xu, J. pharm. Sci. 91(7): 1669-1677, 2002), hydroxyethyl cellulose was proposed as a cytocompatible cross-linker of neutral chitosan-GP gels for cell encapsulation through a proposed mechanism of hydrogen bonding. The present invention is completely distinguished from these previous descriptions, by teaching a method and composition to encapsulate live cells using glyoxal-based cross-linking mechanism of chitosan-GP solutions that results in retention of viable cells in solidified gels.

In summary of prior art, acid-chitosan solutions have been cross-linked with an array of bifunctional cross-linkers with no evidence that these gelling solutions are able to maintain cell viability. Therefore, there is presently a lack of evidence concerning encapsulation of viable cells in chemically cross-linked chitosan gels,

It would be highly desirable to be provided with a new composition for use in medical contexts of tissue repair and regeneration.

### SUMMARY OF THE INVENTION

One aim of the present invention is to provide a biocompatible polymeric liquid solution loaded with cells or biologically active factors, which can solidify and form an implant or film with entrapped or immobilized cells or factors. The solution can thus form a biocompatible solid scaffolding that sustains cell viability, or offers controlled release of bioactive molecules at the injection site. After injection, the implant may give a therapeutic effect from delivered cells, hormones, drugs, DNA, or bulking agent.

In accordance with the present invention, there is provided a composition for immobilizing and encapsulating viable and functional cells or bioactive substances comprising:
a) a liquid polysaccharide solution of isotonic neutral chitosan; and
b) a cross-linking solution consisting of glyoxal-treated hydroxyethyl cellulose, dissolved in physiological media.

The cross-linking solution consists of glyoxal-treated hydroxyethyl cellulose dissolved in physiological media.

The chemical cross-linker is preferably dissolved in physiological media harboring one more more cell nutrients including but not limited to glucose, vitamins, amino acids, and buffering agents as are found in typical cell culture media.

The composition of the present invention may comprise for example:
a) 0.5 to 5.0% by weight chitosan; and
b) 0.0001 - 3 % glyoxal,
and
c) 0.01 to 5.0% by weight glyoxal-treated hydroxyethyl cellulose; and
Wherein said solution form a gel between temperatures of 4°C and 42°C, and more preferably between 20°C and 42°C, said gel providing a physiological environment for maintaining viability of cells.

The composition forms a gel, preferably within seconds to several hours after mixing (a) and (b), and (c).

The chitosan is preferably dissolved in dilute acid and mixed with 1.0 to 2.5% by weight of a salt of polyol consisting of mono-phosphate dibasic salt, such as mono-phosphate dibasic salt of glycerol like glycerol-2-phosphate dibasic salt, sn-glycerol 3-phosphate dibasic salt and L-glycerol-3-phosphate dibasic salt, or mono-sulfate salt.

The chitosan may further be mixed with phosphate buffer and salt.

In one embodiment of the invention, the composition further comprises a biologically active factor. Such factor may be for example selected from the group consisting of cells, a hormones, a drug, DNA, a bulking agent, a growth factors, a DNA, DNA-polymer complexes, liposomes, a pharmacological agent, a metabolic factor, an antibody, a nutritive factor, an angiogenic factor, and a radioisotope.

In one embodiment of the invention, the composition is loaded with cells and more preferably live cells. The cells can be nucleus pulpopus, annulus fibrosis, or a mixture thereof. Alternatively, the cells can be non-human embryonic stem cells or stem cells derived from a tissue selected from the group consisting of bone marrow, adipose, muscle, brain, skin, liver, vascular smooth muscle, endothelium, blood, or placenta. In fact, the cells could also be primary cells, differentiated cells, genetically modified cells, hybridomas, immortalized cells, transformed cells, tissue fragment cells, organelles, or a mixture thereof, nucleated cells, enucleated cells, germ cells, platelet cells, matrix vesicles, cell vesicles, demineralized bone paste, bone chips, cartilage fragments, or cell fragments or tissue fragments, as well as autologous cells, allogeneic cells or xenogeneic cells.

In one embodiment of the invention, the biologically active factor is a cell attachment factor selected from the group consisting of fibrinogen, fibrin, fibronectin, hyaluronic acid, heparin, collagen, polylysine, polyornithine, receptor-binding cyclic peptide, and receptor-binding protein.

The biologically active factor can also be an enzyme, a growth-factor or a growth factor-immobilized substance, as well as a plasmid DNA in the form of liposomes, a lipid complex, a chitosan complex, a poly-lysine complex, a DEAE dextran complex.

In a preferred embodiment, the biologically active factor is a vaccine, either for active or passive immunization. The vaccine can thus comprise an infective viral particle.

The biologically active factor can also be a nutritive or metabolic factor such as a lipid, amino acids, and a co-factor selected from the group consisting of cholesterol, glutamine, glucosamine, ascorbic acid, pyruvate, and lactate.

The biologically active factor can further be at least one element selected from the group consisting of peripheral blood, bone blood, cord blood, a blood product, blood-borne cells, serum, platelets, platelet-rich plasma, fibrinogen, a clotting factor, and a blood-borne enzyme.

In one embodiment of the invention, the biologically active factor is an osteogenic substance such as a member of the bone morphogenetic protein family selected from the group consisting of TGF-β1, BMP-2, BMP-6, BMP-7, or a mixture thereof.

Still in accordance with the present invention, there is provided the use of the composition of the present invention for soft tissue repair, for site-specific delivery of said biologically active factor, for bone repair, for repairing or resurfacing damaged cartilage or for repairing meniscus. In accordance with the present invention, there is also provided the use of the composition of the present invention for the manufacture of a medicament for the various use mentioned herein.

Of course, one skilled in the art provided with the composition of the present invention, and being told that the composition can be used for the various uses mentioned herein will have no difficulty using the composition in a method of treatment. Accordingly, these methods are also included in the present invention.

In the present application, the expression biologically active factors" is meant to include without limitation any biologically active ingredients, cells that have a therapeutic effect, hormones, drugs, DNA, bulking agent, growth factors, DNA, DNA-polymer complexes, liposomes, pharmacological agents, metabolic factors, antibodies, nutritive factors, angiogenic factors, or radioisotopes etc...

### BRIEF DESCRIPTION OF THE DRAWINGS

Fig. 1A illustrates the method used to generate cytocompatible cross-linker by cross-linking hydroxyethyl cellulose with glyoxal;

Fig. 1B illustrates an example of a mechanism of gellation by mixture of glyoxal-cross-linked hydroxyethyl cellulose with chitosan;

Fig. 1C illustrates various methods for preparing cytocompatible cross-linker;

Figs. 2A to 2C illustrate spectral characterization of active and inactive cross-linker prepared by the method illustrated in Fig. 1C;

Figs. 2D to 2G demonstrate gelation over six minutes after mixing;

Fig. 3 illustrates the evolution of G' and G" with time at room temperature (25°C) for a typical cross-linked formulation comprising the successive mixture of 0.12 g chitosan (76%DDA) dissolved in 9 ml 67 mM HCl solution, 0.41 g b-glycerol phosphate dissolved in 1 ml ddH₂0, and 3 to 30 mg water-soluble Spectrum reagent-grade hydroxyethyl cellulose dissolved in 2 ml buffered Ringer's Lactate solution;

Fig. 4A illustrates viability of cells maintained in hydroxyethyl cellulose, or glyoxal cross-linker for over an hour;

Fig 4B shows viability of cells (MTT assay for live cell metabolism) and cell proliferation (Hoechst DNA assay to reflect cell density) in chitosan gels cross-linked with glyoxal or hydroxyethyl cellulose-glyoxal;

Fig. 5A and 5B illustrate viability of various cell types in chitosan gel cross-linked with hydroxyethyl cellulose /aldehyde (Fig. 5A), or glyoxal (Fig. 5B);

Fig. 5C illustrates comparable viability in 2% low melting agarose gel;

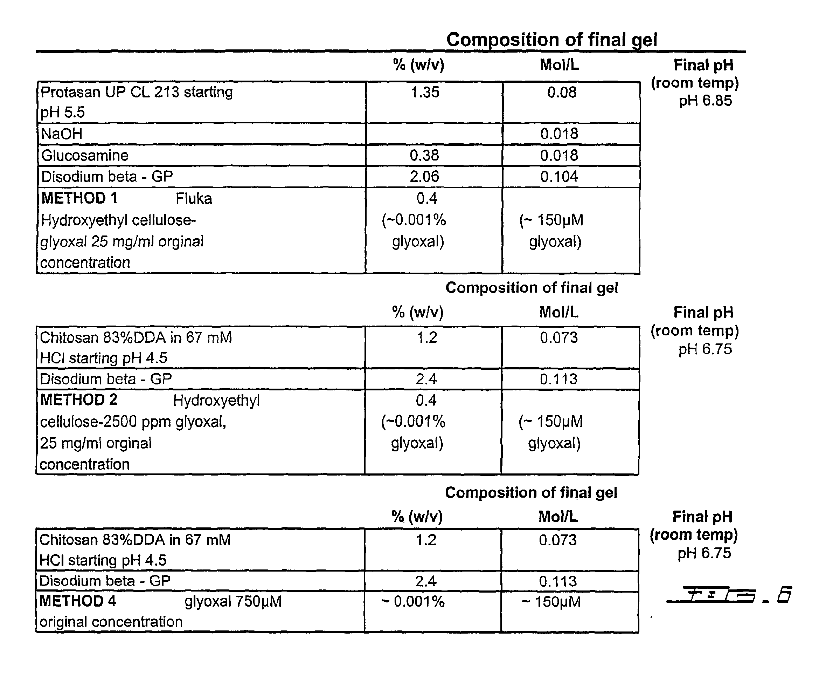

Fig. 6 shows examples of typical compositions of cross-linked cytocompatible chitosan gels using hydroxyethyl cellulose-glyoxal, or glyoxal, used to encapsulate viable cells, in accordance with the present invention;

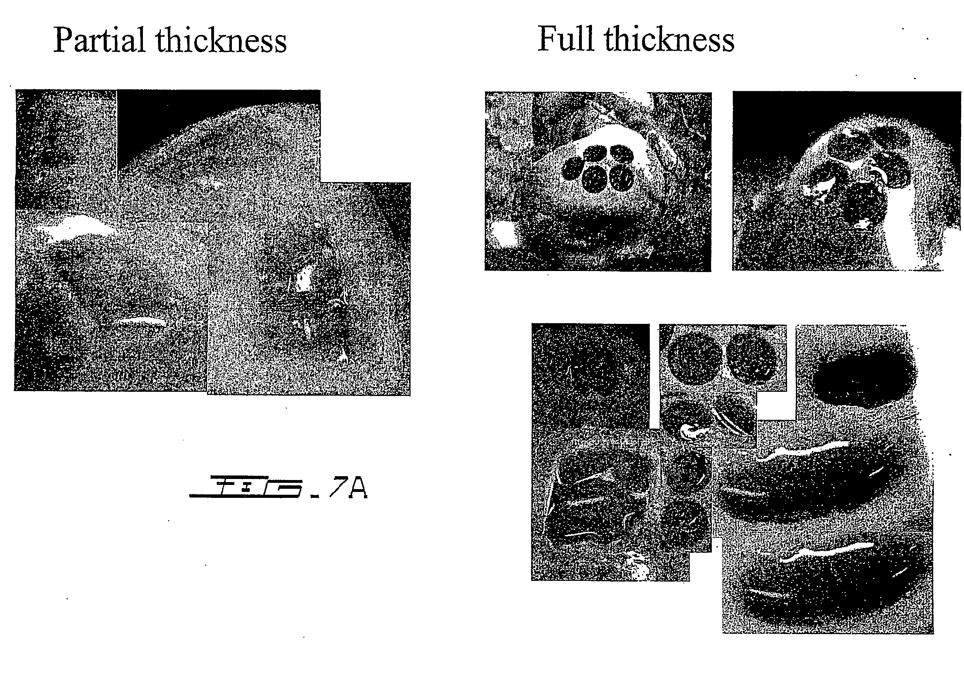

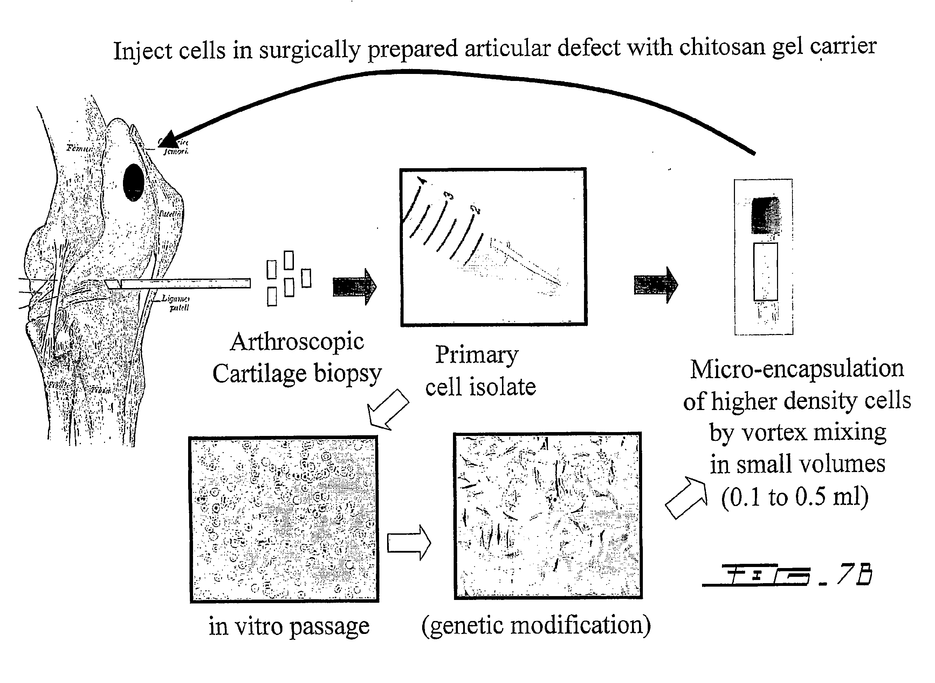

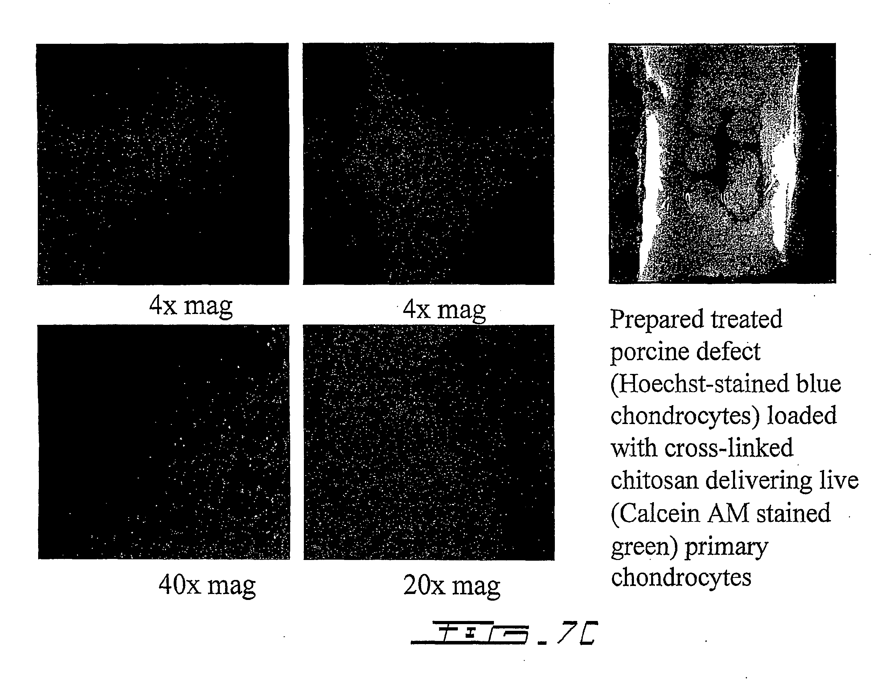

Figs. 7A to 7C show examples of cell delivery applications in cartilage repair using neutral cross-linked chitosan gels using hydroxyethyl cellulose-glyoxal or glyoxal, depending on the application;

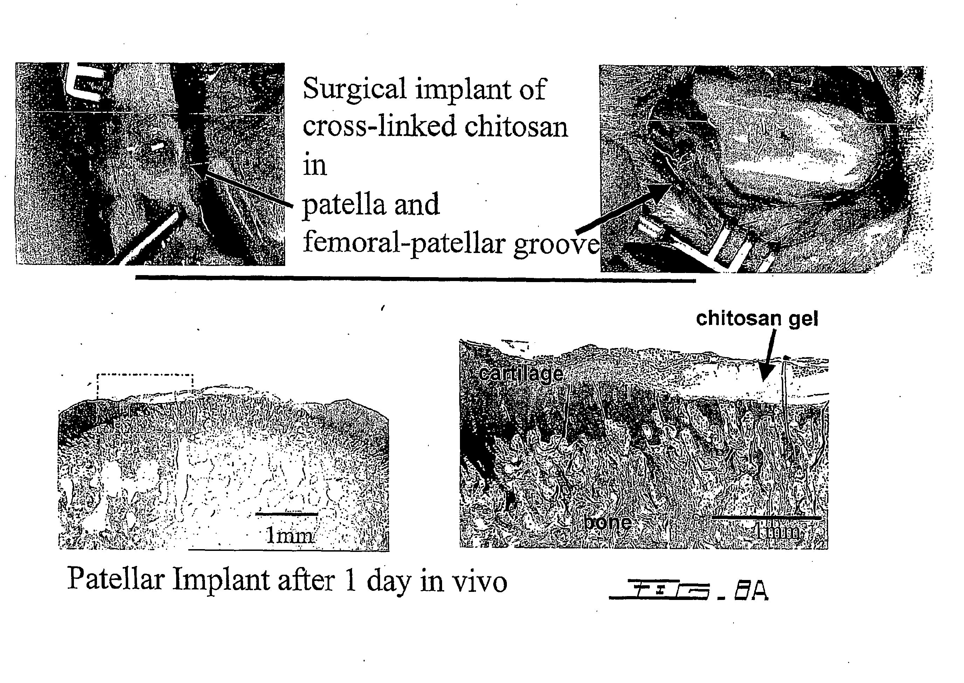

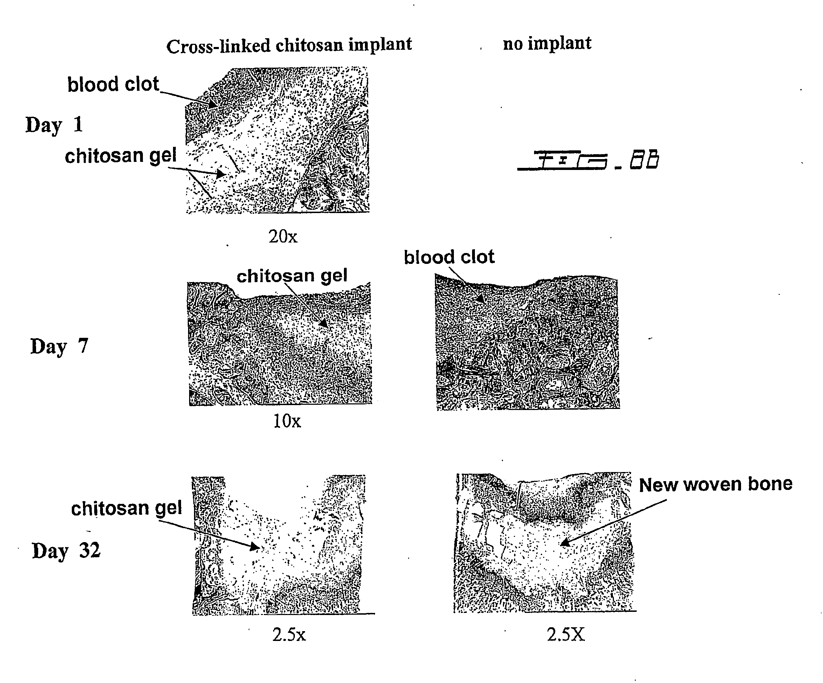

Figs. 8A and 8B shows the persistence cross-linked chitosan gel in vivo, in rabbit articular or osteochondral defects from 1 day, to 30 days post-injection; and

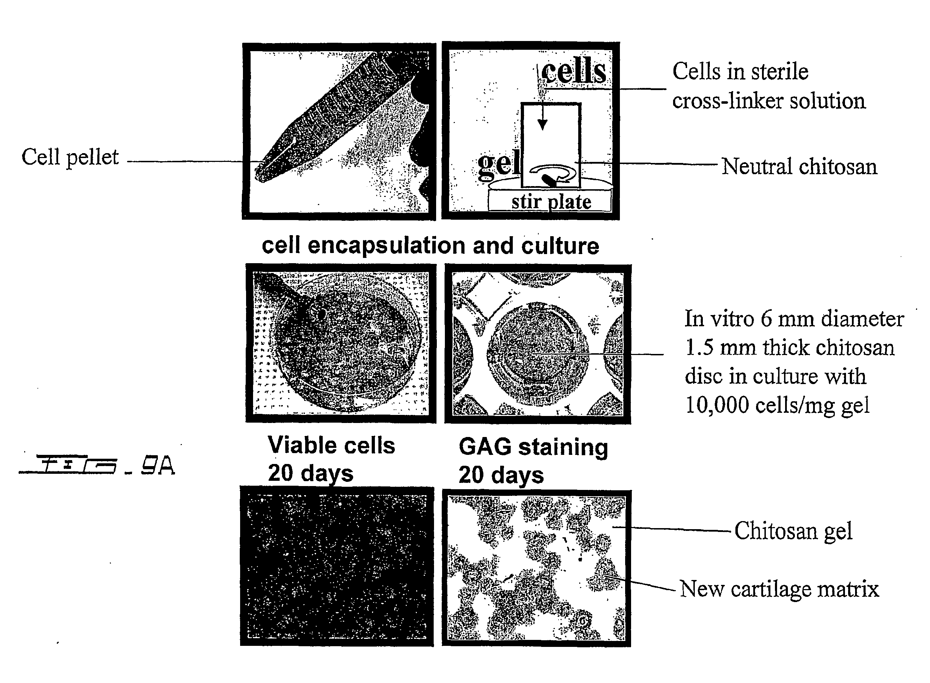

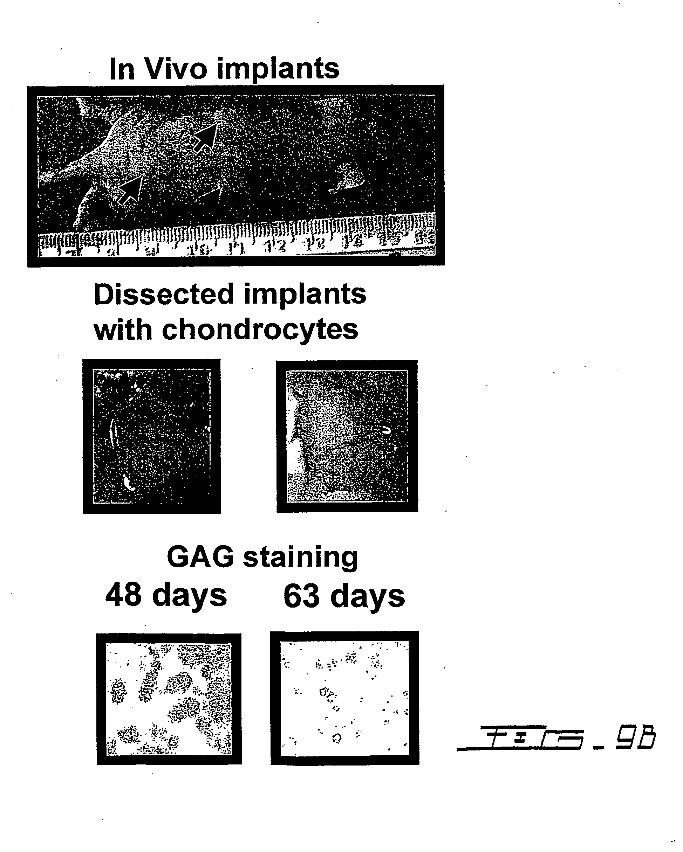

Figs. 9A and 9B show the formation of neocartilage tissue in vitro (Fig. 9A) and in vivo (Fig. 9B) when primary chondrocytes are encapsulated in cross-linked chitosan gel using hydroxyethyl cellulose-glyoxal as the cell carrier.

### DETAINED DESCRIPTION OF THE PREFERRED EMBODIMENT

In accordance with the present invention, there is provided a new procedure of cell immobilization in a polymer matrix of acid-soluble chitosan brought to physiological pH with glycerol phosphate salt, then cross-linked with glyoxal-treated hydroxyethyl cellulose. The bifunctional dialdehyde is presented as a hemi-acetal intermediate conjugated with hydroxyethyl cellulose. This composition maintains high levels of cell viability, provided that the chitosan solution is sterile, and in liquid solution at isotonic and approximately neutral pH. For this purpose, acid-soluble chitosan may be sterilized by autoclave, or the crystalline powder salt form of chitosan sterilized by exposure to UV light prior to dissolving in water. The molecular mass of chitosan may be varied by autoclave-dependent hydrolysis resulting in a reproducible loss in viscosity, prior to adjusting to neutral pH with glycerol phosphate salt. In another embodiment, other phosphate buffers may be used that increase the chitosan solution to pH 6.5 - 6.8, without resulting in chitosan precipitation. The glycerol phosphate salt or phosphate buffer added brings the final osmolarity within physiologically-tolerated limits, or between 200 and 460 mOsm.

The pH dependence of chitosan cross-linking is strictly related to the percentage of free neutral amine groups available to participate in the cross-linking mechanism. Such a proportion of neutral amine-to-protonated amine groups is affected by the deacetylation level of the chitosan used. 95% deacetylated chitosan may be cross-linked at pH 5.0, whereas 80% deacetylated chitosan may only be cross-linked at a higher pH, above 6.0. The most favorable pH used to cross-link chitosan and simultaneously retain cell viability is generally above pH 6.5 at room temperature.

In accordance with the present invention there is provided a method for encapsulating and delivering live cells to a cell culture petri, ex vivo tissue, or in vivo within an implant, wound, organ space, or defect. Further, there is provided a method for co-gellation and sustained release of admixed proteins, such as IGF-1.

Cells are immobilized in neutral chitosan liquid solution with the aid of a cross-linking reagent. In the present embodiment, the cross-linking agent consists of glyoxal mixed with a polymer harboring reactive hydroxyl groups, such as hydroxyethyl ether. The combination of glyoxal-hydroxyethyl cellulose has much reduced toxicity to cells, because the presence of hydroxyethyl cellulose hinders the glyoxal aldehyde groups from reacting with the cell surface. The chitosan amine groups will preferentially attack the glyoxal reactive hydroxyl groups, resulting in a lattice of glyoxal-linked chitosan amine groups with hydroxyethyl cellulose interspersed throughout.

The preferred physiological medium used to suspend the cross-linking agent is a nutrient medium suitable for cell culture, as opposed to simple buffered or unbuffered saline solutions.

To immobilize cells homogenously, a cell pellet is completely resuspended in an aqueous solution of hydroxyethyl cellulose harboring glyoxal, then mixed with a neutral chitosan solution. The resulting mixture may be poured, or injected into the appropriate defect or mold, whereupon solidification occurs. The resulting gel has variable viscoelastisity, adhesivity, and stiffness, depending on the relative amounts of chitosan, glyoxal, and hydroxyethyl cellulose present in the mixture.

The injectable solution may also be used as a bulking agent or tissue sealant.

The present invention also includes, but is not limited to, the example of articular cartilage repair, where delivery of primary and/or passaged chondrocytes with said mixture to an articular cartilage defect will sustain cell viability, and permit proper cell differentiation and the synthesis and assembly of a dense mechanically functional articular cartilage extracellular matrix in situ. The invention includes intervertebral disc repair, where cross-linked gel, or cross-linked gel loaded with matrix-producing cells, is delivered to the damaged disc.

The injectable solution can also be previously mixed with growth factors, DNA, DNA-polymer complexes, liposomes, pharmacological agents, metabolic factors, antibodies, nutritive factors, angiogenic factors, or radioisotopes. To do so, these factors can be mixed with either the neutral chitosan solution, or with the cross-linking hydroxyethyl cellulose glyoxal solution, prior to combining the chitosan and cross-linker.

In another embodiment, the cells may be suspended in a neutral chitosan solution, then mixed into hydroxyethyl cellulose neutral solution, with a range of chitosan/hydroxyethyl cellulose/cross-linker proportional volumes.

The glyoxal-treated hydroxyethyl cellulose needed to cross-link chitosan is preferably obtained by one of several methods from commercially available medium viscosity non-pharmaceutical grade hydroxyethyl cellulose. In routine industrial processing, hydroxyethyl cellulose is surface-treated with glyoxal to induce cross-links. The cross-linked hydroxyethyl cellulose is slow to dissolve in water, and therefore has reduced lumping. It is in these preparations that active chitosan cross-linker may be obtained. Pharmaceutical-grade hydroxyethyl cellulose, which has been treated to remove glyoxal, cannot be used to prepare active chitosan cross-linker.

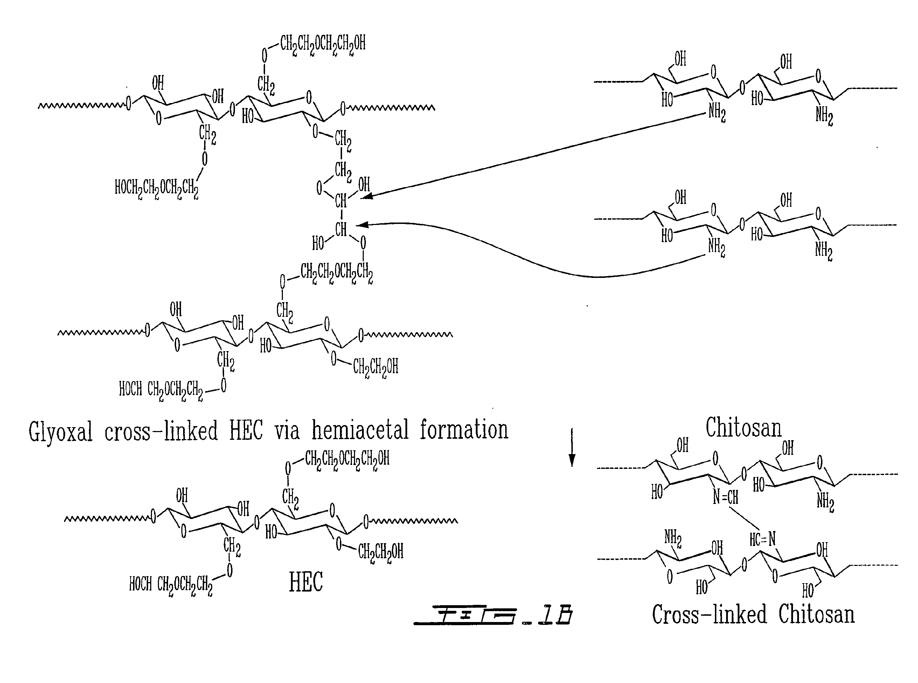

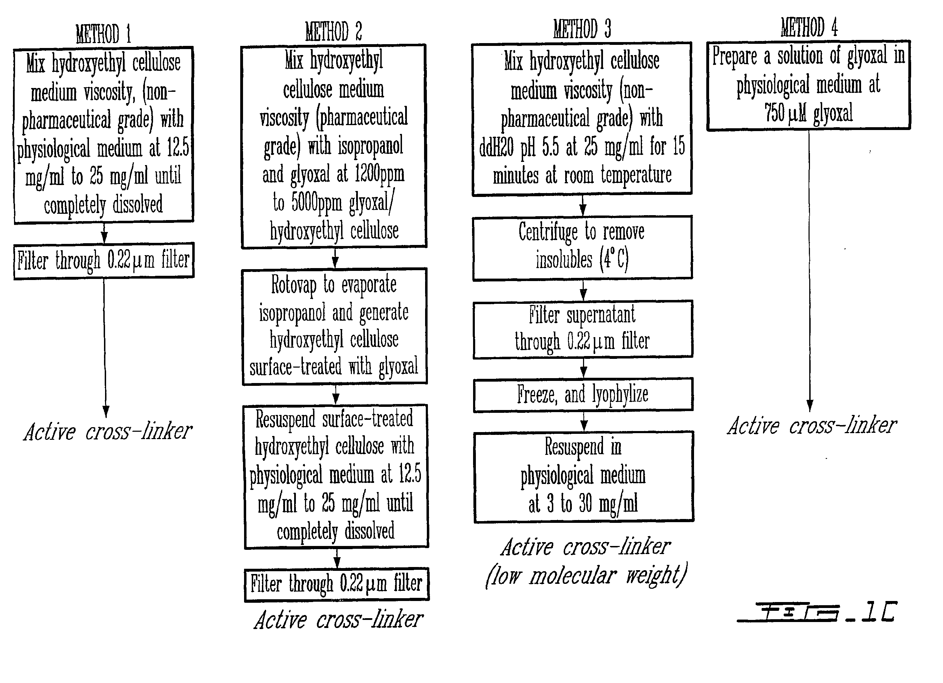

Several methods may be used to prepare cytocompatible cross-linker. By one method, certain types of medium viscosity hydroxyethyl cellulose (Fluka) can be dissolved completely to 25 mg/ml in aqueous solution at physiological pH. In one method (Method 4), a solution of 40% glyoxal (8.76M) is diluted to 750µM in physiological medium. The resulting solution may be used as active cross-linker by mixing 1 part with 4 parts neutral chitosan., then sterilized by filtration through a 0.22mm filter (method 1, Fig. 1C). Fig. 1C illustrates method 1, wherein hydroxyethyl cellulose of medium viscosity (3,400 cPa), non-pharmaceutical grade, from Fluka having slow dissolving time in water, has been cross-linked with glyoxal to retard the rate of hydration and to minimize lumping. If dissolved completely at 12.5 mg/ml to 25 mg/ml in physiological medium, the resulting solution may be sterile-filtered through a 0.22µm filter, and used as active cross-linker by mixing 1 part filtered hydroxyethyl cellulose with 4 parts 1.5% neutral chitosan.

By another method, pharmaceutical grade hydroxyethyl cellulose is surface-treated with glyoxal and dried prior to dissolving in physiological media and filter sterilization (method 2, Fig. 1C). In Method 2 illustrated in Fig. 1C, hydroxyethyl cellulose of medium or low viscosity (pharmaceutical grade: below 500 ppm glyoxal or no glyoxal), is combined with 2500ppm to 3500 ppm glyoxal in a polar solvent, and dried to generate hydroxyethyl cellulose surface treated with glyoxal. The resulting powder may be dissolved at 25 mg/ml in physiological medium, sterile-filtered, and used as an active cross-linker as described for Method 1 above. By another method, hydroxyethyl cellulose is mixed at 25 mg/ml with ddH₂0 for 15 minutes at room temperature, where the particles are resistant to water solubilization.

In method 3 of Fig. 1C, hydroxyethyl cellulose of medium viscosity, non-pharmaceutical grade, from Spectrum or Fluka, both have slow dissolving time in water. The water-soluble hydroxyethyl cellulose fraction is recovered, lyophilized, and the resulting solid resuspended in aqueous solution, which is physiological in pH and osmolarity (method 3, Fig. 1C). If the hydroxyethyl cellulose is mixed for 15 minutes in water, the aqueous phase which contains small molecular weight hydroxyethyl cellulose and in addition reactive glyoxal may be recovered by centrifuging out insolubles, and filtering through a 0.22µm filter. The resulting solution may be concentrated and used to cross-link neutral chitosan by mixing 1 part (1mg/ml to 30 mg/ml) water-soluble hydroxyethyl cellulose with 4 parts neutral chitosan.

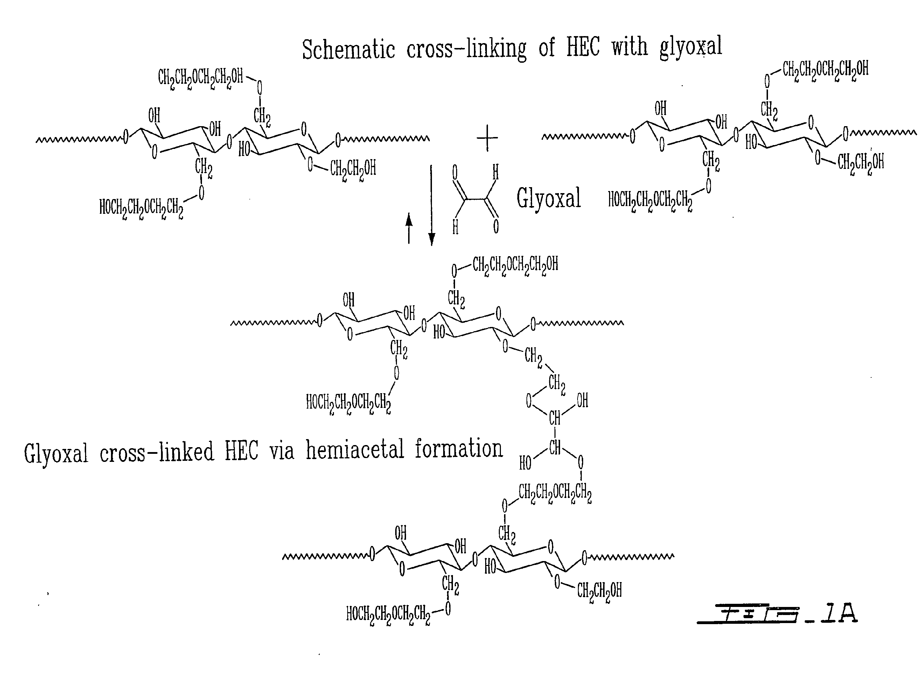

Alternatively, glyoxal may also be diluted to that concentration present in surface-treated hydroxyethyl cellulose (near 0.001 %) in physiological medium and rendered filter-sterile (method 4, Fig. 1D). In method 4 of Fig. 1C, a solution of 40% glyoxal (8.76M) is diluted to 750µM in physiological medium. The resulting solution may be used as active cross-linker by mixing 1 part with 4 parts neutral chitosan. Some commercial hydroxyethyl cellulose powders will form a gel when dissolved completely at 25 mg/ml (Spectrum, Hercules). In this event, reactive cross-linker may only be obtained if the hydroxyethyl cellulose has been cross-linked with glyoxal, or another similar reagent, and if water-soluble material (containing low molecular weight cross-linked hydroxyethyl cellulose) can be extracted from slowly dissolving particles. Regardless of the method used to prepare the hydroxyethyl cellulose solution, once hydrated, the solution shall be protected from hydrolysis or conformational changes by frozen storage.

Active cross-linker can be purified from a low molecular weight fraction (below 1000 Da) of water-soluble hydroxyethyl cellulose from Spectrum. However, the more purified the cross-linker becomes, the more toxic an effect it has on cells. Therefore, the optimal cross-linking conditions for cell viability are those which use a cross-linking agent in the presence of an alternative polymer upon which the cross-linker may react, but which has less affinity for the cross-linker than does chitosan neutral amine groups. When the apparent toxic effect is due to co-purifying contaminants from the initial hydroxyethyl preparation, this toxicity may be partly avoided by using pure glyoxal at highly dilute concentrations in media.

The hydroxyethyl cellulose solution used to cross-link the chitosan-glycerol phosphate solution is preferably 0.5% to 98% the bulk mass of chitosan present in liquid solution. The solution is preferentially sterilized by filtration through a 0.22mm filter. To those skilled in the art, it becomes obvious that any multifunctional reactive compound which may form reversible cross-links with a suitable polymer carrier could be used as a reduced toxicity, cytocompatible cross-linker for any amine-containing polymer, to entrap cells or bioactive molecules that are sensitive to incubation with the multifunctional compound alone.

Once prepared, the concentrated water-soluble hydroxyethyl cellulose is suspended in a physiological buffered solution, such as phosphate-buffered saline, Ringer's buffered lactate, cell culture medium such as Dulbecco's modified Eagle Medium, sterile 0.9% saline, or other preparations of cytocompatible nutrient medias used in cell culture. For delivery of some bioactive substances, chemicals, liposomes, radioisotopes, or pharmaceutical agents, the hydroxyethyl cellulose can be suspended in water or other conditions in order to combine completely with these materials prior to mixing with chitosan. For instances such as this, the chitosan does not necessarily need to be rendered to physiological pH, but instead, 95% deacetylated chitosan may be dissolved in a minimum amount of acid, and used at a pH of 4.0 to 5.5.

The present invention demonstrates that the gellation mechanism of neutral chitosan solutions using hydroxyethyl cellulose cross-linker may only occur when the hydroxyethyl cellulose solution has been previously combined with glyoxal in a surface treatment during routine large-scale industrial preparation. The present invention furthermore demonstrates that the cross-linking activity of hydroxyethyl cellulose is lost when glyoxal is eliminated by dialysis, or by other specific treatments used to remove glyoxal to generate a pharmaceutical grade product. It is shown in the present invention that at low concentrations (below 0.01%) glyoxal may be used to cross-link neutral chitosan solutions while maintaining cell viability, however initial cell metabolism (as an index of cell viability) of cells encapsulated in such glyoxal cross-linked gels is lower than that of cells encapsulated with hydroxyethyl cellulose-glyoxal. The kinetics of gellation shown in the examples of this invention are compatible with clinical use, from seconds to one hour, and permit the gellation and retention of gel with or without cells and/or medically active agents in a body cavity, petri dish, or open wound.

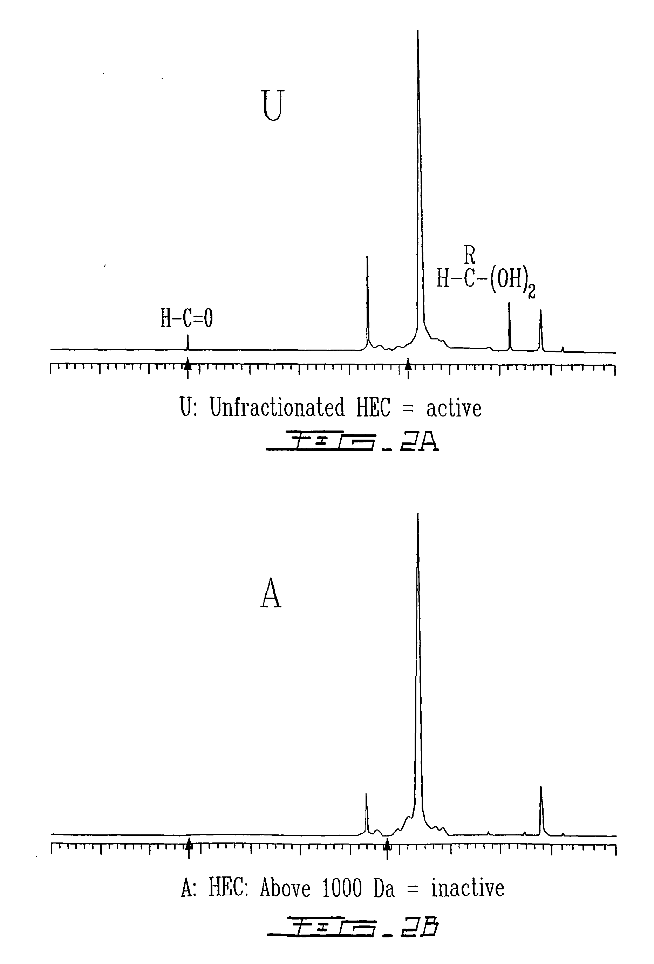

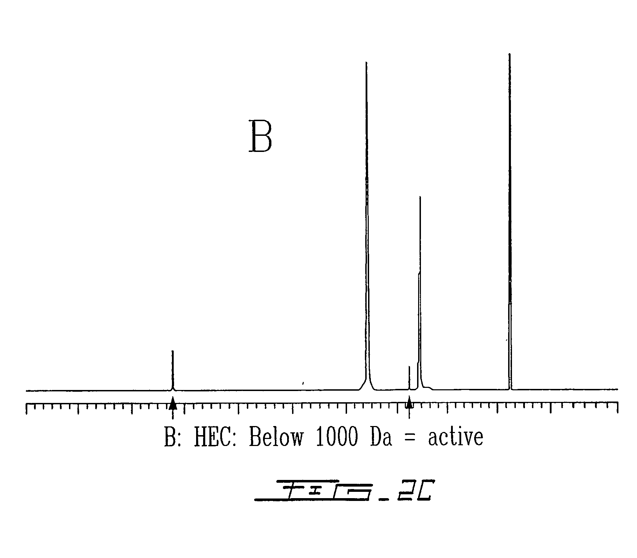

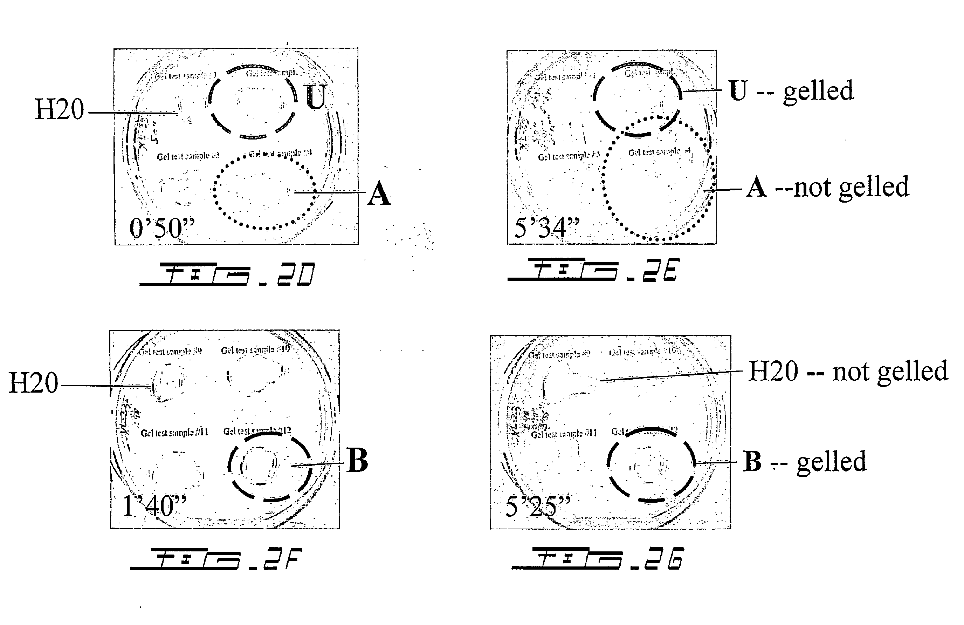

In Figs. 2A to 2G, cross-linking activity correlates with those hydroxyethyl cellulose fractions containing aldehyde-like 1H-NMR peaks (peak at 8.3ppm) and hemiacetal peaks (3.8ppm). Cross-linker was prepared according to method 3 in Fig. 1C, and subsequently fractionated by ultrafiltration to collect fractions above and below 1000 Da. Each fraction was submitted to NMR analysis (upper panels). Each of the fractions was suspended at 7.5 mg/ml in ddH₂0, and mixed with neutral chitosan at 1 part hydroxyethyl cellulose fraction, 5 parts 1.5% neutral chitosan solution. The samples were deposited on a plastic petri, and tilted at timed intervals to demonstrate gellation (lower panels). Unfractionated, and the low molecular mass fraction (below 1000 Da) induced rapid gellation of chitosan within 5 minutes. Dialysed hydroxyethyl cellulose failed to gellify the chitosan, indicating that hydroxyethyl cellulose is not sufficient to cross-link chitosan under the test conditions. Both active cross-linking samples harbor peaks consistent with the presence of an aldehyde (8.3 ppm) and hemiacetal (3.8 ppm).

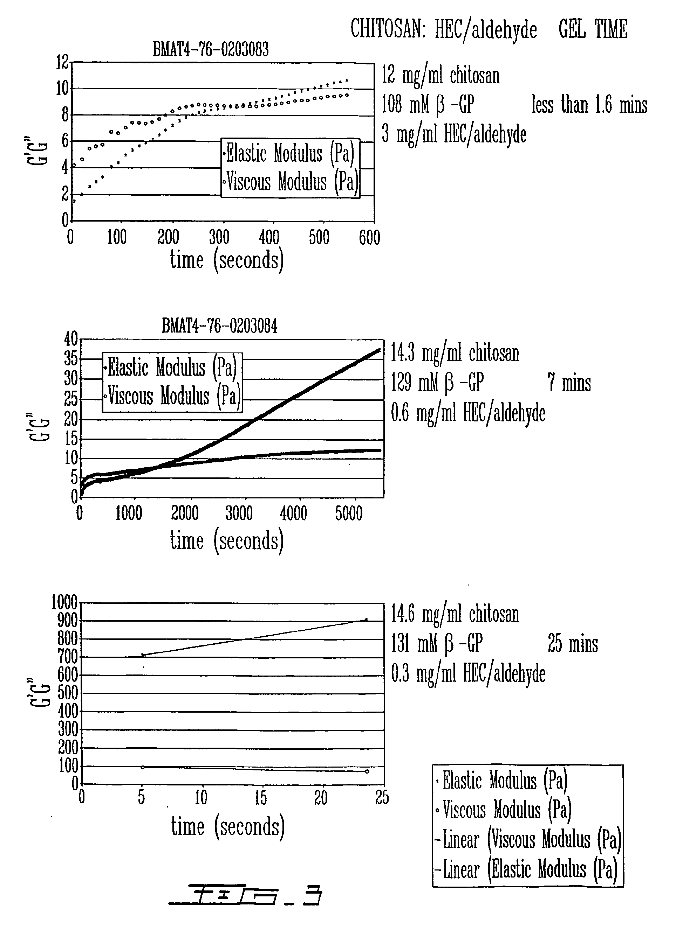

In Fig. 3, t=0 occurs 1.6 minutes after mixing. The results show a dose-dependency between gellation time, and hydroxyethyl cellulose-glyoxal concentration.

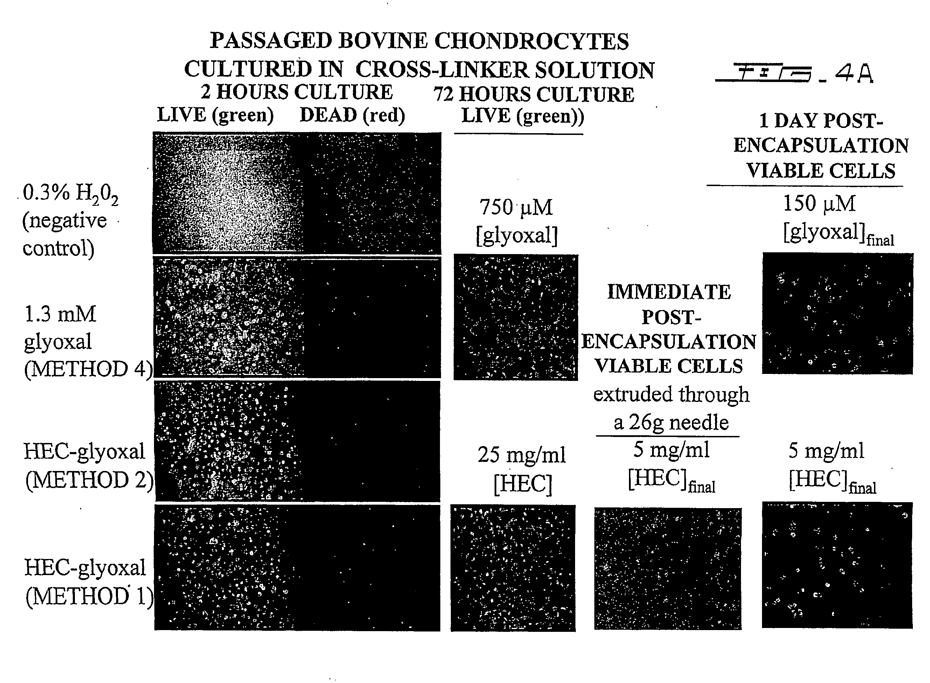

In Fig. 4A, high viability is maintained after encapsulation in cross-linked chitosan with glyoxal, or hydroxyethyl cellulose-glyoxal. Fig. 4A illustrates that the active hydroxyethyl cellulose-cross linker is cytocompatible. Cells incubated up to 72 hours in active cross-linker remain over 95% viable. Cells incubated in 0.3% peroxide for the same time period are 100% non-viable. After mixing with chitosan and injecting through a syringe with a 26-gauge needle, encapsulated cells in solid gel remain over 95% viable. After mixing with chitosan and pouring into a petri, encapsulated cells in solid gel remain over 95% viable after 1 day of culture.

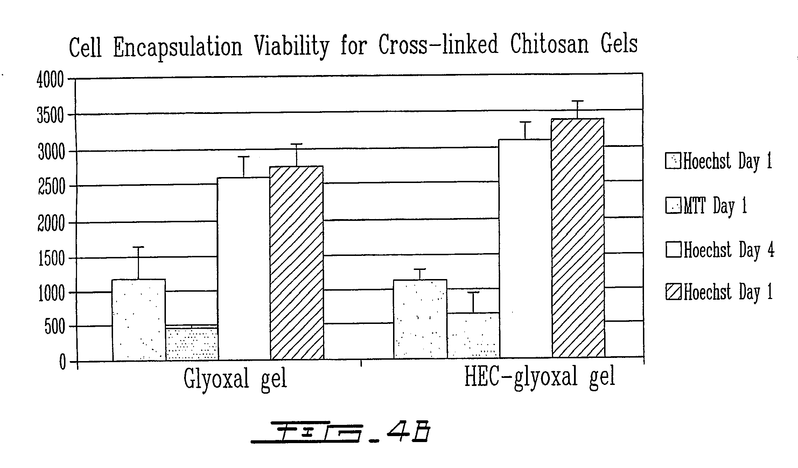

In Fig. 4B, as shown by MTT assay on day 1 encapsulated cells, hydroxyethyl cellulose offers additional protection to cells immediately post-encapsulation. Cells encapsulated in chitosan gel using either glyoxal or hydroxyethyl cellulose-glyoxal are viable after encapsulation and proliferate in the gel. Cells show greater viability as measured by a metabolic MTT assay, at 1 day post-encapsulation when the active cross-linker is hydroxyethyl cellulose-glyoxal, compared to glyoxal cross-linker.

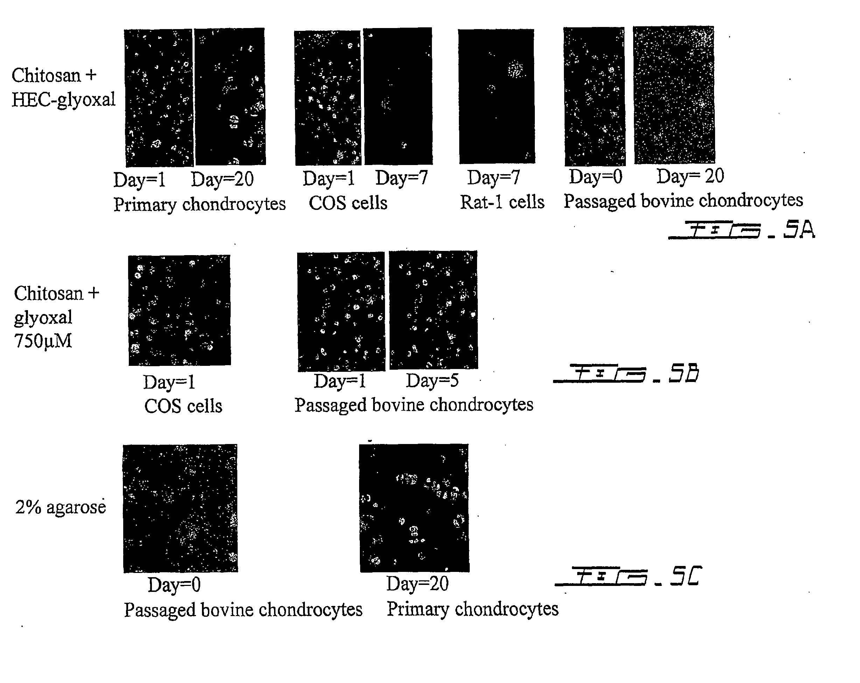

In Figs. 5A to 5C, green is indicative of live cells and red is indicative of dead cells. As can be noted, Fig. 5A shows the persistence of a range of viable cell types cast in chitosan gels cross-linked with hydroxyethyl cellulose-glyoxal, including fibroblast cell lines Rat-1, COS, bovine primary chondrocytes, and bovine passaged chondrocytes at casting and after culture. Fig. 5B shows persistence of COS cell and passaged bovine chondrocyte cell viability in glyoxal cross-linked chitosan gels. Fig. 5C shows comparable viability of primary and passaged bovine chondrocytes cast in 2% low melting point agarose.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A composition for immobilizing and encapsulating viable and functional cells or bioactive substances comprising:
a) a liquid polysaccharide solution of isotonic neutral chitosan; and
b) a cross-linking solution consisting of glyoxal-treated hydroxyethyl cellulose dissolved in a physiological medium.

2. The composition of claim 1, wherein the composition comprises:
a) 0.5 to 5.0% by weight chitosan; and
b) 0.01 to 5.0% by weight glyoxal-treated hydroxyethyl cellulose,
Wherein said solution form a gel between temperatures of 4° C and 42° C, said gel providing a physiological environment for maintaining viability of cells.

3. The composition of claim 2, further comprising:
c) 0.0001-3 % glyoxal,

4. The composition of claim 2, wherein the composition form a gel within seconds to several hours after mixing (a) and (b).

5. The composition of claim 3, wherein the composition form a gel within seconds to several hours after mixing (a), (b) and (c).

6. The composition of claim 2, wherein the solution form a gel between temperatures of 20° C and 42° C.

7. The composition of claim 1, wherein the chitosan is dissolved in dilute acid and mixed with 1.0 to 2.5% by weight of a salt of polyol consisting of mono-phosphate dibasic salt, or mono-sulfate salt.

8. The composition of claim 7, wherein said mono-phosphate dibasic salt is mono-phosphate dibasic salt of glycerol.

9. The composition of claim 7, wherein mono-phosphate dibasic salt of glycerol is selected from the group consisting of glycerol-2-phosphate dibasic salt, sn-glycerol 3-phosphate dibasic salt and L-glycerol-3- phosphate dibasic salt.

10. The composition of claim 1, wherein chitosan is further mixed with phosphate buffer and salt.

11. The composition of claim 1, further comprising a biologically active factor.

12. The composition of claim 11, wherein the biologically active factor is selected from the group consisting of cells, a hormones, a drug, DNA, a bulking agent, a growth factors, a DNA, DNA-polymer complex, liposomes, a pharmacological agent, a metabolic factor, an antibody, a nutritive factor, an angiogenic factor, and a radioisotope.

13. The composition of claim 12, wherein said cells are live cells.

14. The composition of claim 12, wherein the cells are nucleus pulpopus, annulus fibrosis, or a mixture thereof.

15. The composition of claim 12, wherein the cells are non-human embryonic stem cells or stem cells derived from a tissue selected from the group consisting of bone marrow, adipose, muscle, brain, skin, liver, vascular smooth muscle, endothelium, blood, or placenta.

16. The composition of claim 12, wherein the cells are primary cells, differentiated cells, genetically modified cells, hybridomas, immortalized cells, transformed cells, tissue fragment cells, organelles, or a mixture thereof, nucleated cells, enucleated cells, germ cells, platelet cells, matrix vesicles, cell vesicles, demineralized bone paste, bone chips, cartilage fragments, or cell fragments or tissue fragments.

17. The composition of claim 12, wherein the cells are autologous cells, allogeneic cells or xenogeneic cells.

18. The composition of claim 12, wherein the biologically active factor is a cell attachment factor selected from the group consisting of fibrinogen, fibrin, fibronectin, hyaluronic acid, heparin, collagen, polylysine, polyornithine, receptor-binding cyclic peptide, receptor-binding protein.

19. The composition of claim 12, wherein the biologically active factor is an enzyme, a growth-factor or a growth factor-immobilized substance.

20. The composition of claim 12, wherein the biologically active factor is a plasmid DNA in the form of liposomes, a lipid complex, a chitosan complex, a poly-lysine complex, a DEAE dextran complex.

21. The composition of claim 12, wherein the biologically active factor is a vaccine.

22. The composition of claim 21, wherein the vaccine comprises an infective viral particle.

23. The composition of claim 12, wherein the biologically active factor is a nutritive or metabolic factor.

24. The composition of claim 23, wherein the a nutritive or metabolic factor is a lipid, amino acids, and a co-factor selected from the group consisting of cholesterol, glutamin, glucosamine, ascorbic acid, pyruvate, and lactate.

25. The composition of claim 12, wherein the biologically active factor is at least one element selected from the group consisting of peripheral blood, bone blood, cord blood, a blood product, blood-borne cells, serum, platelets, platelet-rich plasma, fibrinogen, a clotting factor, and a blood- borne enzyme.

26. The composition of claim 12, wherein the biologically active factor is an osteogenic substance.

27. The composition of claim 26, wherein the osteogenic substance is a member of the bone morphogenetic protein family selected from the group consisting of TGF-β1, BMP-2, BMP-6, BMP-7, or a mixture thereof.

28. The composition in claim 1, wherein hydroxyl-containing polymer is polyvinyl alcohol, dextran, linked with a bifunctional reactive aldehyde.

29. Use of the composition of any one of claims 1 to 28 for the manufacture of a medicament for soft tissue repair.

30. Use of the composition of claim 12 or 13 for the manufacture of a medicament for site-specific delivery of said biologically active factor.

31. Use of the composition of any one of claims 1 to 28 for the manufacture of a medicament for bone repair.

32. Use of the composition of any one of claims 1 to 28 for the manufacture of a medicament for repairing or resurfacing damaged cartilage.

33. Use of the composition of any one of claims 1 to 28 for the manufacture of a medicament for repairing meniscus.

34. The composition of claim 1, where the physiological medium comprises cell nutrients selected from the group consisting of glucose, amino acids, and vitamins, or a combination thereof, at isotonic and neutral pH.

## Patentansprüche

1. Zusammensetzung zum Immobilisieren und Einkapseln von lebensfähigen und funktionellen Zellen oder bioaktiven Substanzen, umfassend:
a) eine flüssige Polysaccharidlösung von isotonischem neutralem Chitosan; und
b) eine Quervernetzungslösung bestehend aus glyoxal-behandelter Hydroxyethylcellulose gelöst in einem physiologischen Medium.

2. Zusammensetzung nach Anspruch 1, wobei die Zusammensetzung umfasst:
a) 0,5 bis 5,0 Gew.% Chitosan; und
b) 0,01 bis 5,0 Gew.% glyoxal-behandelte Hydroxyethylcellulose,
wobei die Lösung ein Gel zwischen Temperaturen von 4 °C und 42 °C bildet, und das Gel eine physiologische Umgebung zur Aufrechterhaltung der Lebensfähigkeit von Zellen bereitstellt.

3. Zusammensetzung nach Anspruch 2, ferner umfassend:
c) 0,0001 bis 3 % Glyoxal.

4. Zusammensetzung nach Anspruch 2, wobei die Zusammensetzung ein Gel innerhalb von Sekunden bis mehrere Stunden nach dem Mischen von (a) und (b) bildet.

5. Zusammensetzung nach Anspruch 3, wobei die Zusammensetzung ein Gel innerhalb von Sekunden bis mehrere Stunden nach dem Mischen von (a), (b) und (c) bildet.

6. Zusammensetzung nach Anspruch 2, wobei die Lösung ein Gel zwischen Temperaturen von 20 °C und 42 °C bildet.

7. Zusammensetzung nach Anspruch 1, wobei das Chitosan in verdünnter Säure gelöst und mit 1,0 bis 2,5 Gew.% eines Salzes von Polyol bestehend aus dibasischem Monophosphatsalz oder Monosulfatsalz gemischt ist.

8. Zusammensetzung nach Anspruch 7, wobei das dibasische Monophosphatsalz dibasisches Monophosphatsalz von Glycerol ist.

9. Zusammensetzung nach Anspruch 7, wobei das dibasische Monophosphaltsalz von Glycerol aus der Gruppe bestehend aus dibasischem Glycerol-2-phosphatsalz, dibasischem sn-Glycerol-3-phosphatsalt und dibasischem L-Glycerol-3-phosphatsalz ausgewählt ist.

10. Zusammensetzung nach Anspruch 1, wobei Chitosan ferner mit Phosphatpuffer und Salz gemischt ist.

11. Zusammensetzung nach Anspruch 1, ferner umfassend einen biologisch aktiven Faktor.

12. Zusammensetzung nach Anspruch 11, wobei der biologisch aktive Faktor aus der Gruppe bestehend aus Zellen, Hormonen, einem Arzneimittel, DNA, einem Füllstoff, Wachstumsfaktoren, einer DNA, DNA-Polymerkomplex, Liposomen, einem pharmakologischen Mittel, einem Stoffwechselfaktor, einem Antikörper, einem Ernährungsfaktor, einem angiogenen Faktor und einem Radioisotop ausgewählt ist.

13. Zusammensetzung nach Anspruch 12, wobei es sich bei den Zellen um lebende Zellen handelt.

14. Zusammensetzung nach Anspruch 12, wobei es sich bei den Zellen um Nucleus pulpopus, Annulus fibrosis oder eine Mischung davon handelt.

15. Zusammensetzung nach Anspruch 12, wobei die Zellen nichthumane embryonale Stammzellen oder Stammzellen sind, die von einem Gewebe stammen, das aus der Gruppe bestehend aus Knochenmark, Fett, Muskel, Hirn, Haut, Leber, glattem Gefäßmuskel, Endothel, Blut oder Plazenta ausgewählt ist.

16. Zusammensetzung nach Anspruch 12, wobei es sich bei den Zellen um Primärzellen, differenzierte Zellen, genetisch modifizierte Zellen, Hybridome, sich unbegrenzt vermehrende Zellen, transformierte Zellen, Gewebefragmentzellen, Organellen oder eine Mischung davon, kernhaltige Zellen, kernlose Zellen, Keimzellen, Thrombozytenzellen, Matrixvesikel, Zellvesikel, entmineralisierte Knochenmasse, Knochensplitter, Knorpelfragmente oder Zellfragmente oder Gewebefragmente handelt.

17. Zusammensetzung nach Anspruch 12, wobei es sich bei den Zellen um autologe Zellen, allogene Zellen oder xenogene Zellen handelt.

18. Zusammensetzung nach Anspruch 12, wobei der biologisch aktive Faktor ein Zellbindungsfaktor ist, der aus der Gruppe bestehend aus Fibrinogen, Fibrin, Fibronectin, Hyaluronsäure, Heparin, Collagen, Polylysin, Polyornithin, rezeptorbindendem zyklischem Peptid, rezeptorbindendem Protein ausgewählt ist.

19. Zusammensetzung nach Anspruch 12, wobei der biologisch aktive Faktor ein Enzym, ein Wachstumsfaktor oder eine Wachstumstaktorimmobilisierte Substanz ist.

20. Zusammensetzung nach Anspruch 12, wobei der biologisch aktive Faktor eine Plasmid-DNA in Form von Liposomen, einem Lipidkomplex, einem Chitosankomplex, einem Polylysinkomplex, einem DEAE-Dextrankomplex ist.

21. Zusammensetzung nach Anspruch 12, wobei der biologisch aktive Faktor ein Impfstoff ist.

22. Zusammensetzung nach Anspruch 21, wobei der Impfstoff ein infektiöses Viruspartikel umfasst.

23. Zusammensetzung nach Anspruch 12, wobei der biologisch aktive Faktor ein Ernährungs- oder Stoffwechselfaktor ist.

24. Zusammensetzung nach Anspruch 23, wobei es sich bei dem Ernährungs- oder Stoffwechselfaktor um ein Lipid, Aminosäuren und einen Cofaktor handelt, der aus der Gruppe bestehend aus Cholesterin, Glutamin, Glucosamin, Ascorbinsäure, Pyruvat und Lactat ausgewählt ist.

25. Zusammensetzung nach 12, wobei der biologisch aktive Faktor wenigstens ein Element ist, das aus der Gruppe bestehend aus peripherem Blut, Knochenblut, Nabelblut, einem Blutprodukt, hämatogenen Zellen, Serum, Thrombozyten, thrombozytenreichem Plasma, Fibrinogen, einem Gerinnungsfaktor und einem hämatogenen Enzym ausgewählt ist.

26. Zusammensetzung nach Anspruch 12, wobei der biologisch aktive Faktor eine osteogene Substanz ist.

27. Zusammensetzung nach Anspruch 26, wobei die osteogene Substanz ein Mitglied der morphogenetischen Knochenproteinfamilie ist, die aus der Gruppe bestehend aus TGF-01, BMP2, BMP-6, BMP-7 oder einer Mischung davon ausgewählt ist.

28. Zusammensetzung nach Anspruch 1, wobei hydroxylhaltiges Polymer Polyvinylalkohol, Dextran, verbunden mit einem bifunktionellen reaktiven Aldehyd, ist.

29. Verwendung der Zusammensetzung nach einem der Ansprüche 1 bis 28 zur Herstellung eines Medikaments zur Weichgeweberekor_struktion.

30. Verwendung der Zusammensetzung nach Anspruch 12 oder 13 zur Herstellung eines Medikaments zur ortsspezifischen Verabreichung des biologisch aktiven Faktors.

31. Verwendung der Zusammensetzung nach einem der Ansprüche 1 bis 28 zur Herstellung eines Medikaments zur Knochenrekonstrukticn.

32. Verwendung der Zusammensetzung nach einem der Ansprüche 1 bis 28 zur Herstellung eines Medikaments zur Rekonstruktion oder zum Oberflächenersatz eines geschädigten Knorpels.

33. Verwendung der Zusammensetzung nach einem der Ansprüche 1 bis 28 zur Herstellung eines Medikaments zur Meniskusrekonstruktion.

34. Zusammensetzung nach Anspruch 1, wobei das physiologische Medium Zellnährstoffe umfasst, die aus der Gruppe bestehend aus Glucose, Aminosäuren und Vitaminen oder einer Kombination davon, bei isotonischem und neutralem pH, ausgewählt sind.

## Revendications

1. Composition pour immobiliser et encapsuler des cellules viables et fonctionnelles ou des substances bioactives comprenant :
a) une solution de polysaccharide liquide de chitosan neutre isotonique ; et
b) une solution de réticulation consistant en de l'hydroxyéthyl cellulose traitée par du glyoxal dissoute dans un milieu physiologique.

2. Composition selon la revendication 1, dans laquelle la composition comprend :
a) 0,5 à 5,0 % en poids de chitosan ; et
b) 0,01 à 5,0 % en poids d'hydroxyéthyl cellulose traitée par du glyoxal,
dans laquelle ladite solution forme un gel entre les températures de 4 °C et 42 °C, ledit gel formant un environnement physiologique pour maintenir la viabilité des cellules.

3. Composition selon la revendication 2, comprenant en outre :
c) 0,0001 à 3 % de glyoxal.

4. Composition selon la revendication 2, dans laquelle la composition forme un gel en plusieurs secondes à plusieurs heures après le mélange de (a) et (b) .

5. Composition selon la revendication 3, dans laquelle la composition forme un gel en plusieurs secondes à plusieurs heures après le mélange de (a), (b) et (c).

6. Composition selon la revendication 2, dans laquelle la solution forme un gel entre les températures de 20 °C et 42 °C.

7. Composition selon la revendication 1, dans laquelle le chitosan est dissous dans un acide dilué et mélangé avec 1,0 à 2,5 % en poids d'un sel de polyol consistant en un sel dibasique de monophosphate, ou d'un sel de monosulfate.

8. Composition selon la revendication 7, dans laquelle ledit sel dibasique de monophosphate est le sel dibasique de monophosphate de glycérol.

9. Composition selon la revendication 7, dans laquelle le sel dibasique de monophosphate de glycérol est choisi dans le groupe consistant en le sel dibasique de glycérol-2-phosphate, le sel dibasique de sn-glycérol-3-phosphate et le sel dibasique de L-glycérol-3-phosphate.

10. Composition selon la revendication 1, dans laquelle le chitosan est en outre mélangé avec un tampon de phosphate et un sel.

11. Composition selon la revendication 1, comprenant en outre un facteur biologiquement actif.

12. Composition selon la revendication 11, dans laquelle le facteur biologiquement actif est choisi dans le groupe consistant en des cellules, une hormone, un médicament, de l'ADN, un diluant, un facteur de croissance, un ADN, un complexe ADN-polymère, des liposomes, un agent pharmacologique, un facteur métabolique, un anticorps, un facteur nutritif, un facteur angiogénique et un radio-isotope.

13. Composition selon la revendication 12, dans laquelle lesdites cellules sont des cellules vivantes.

14. Composition selon la revendication 12, dans laquelle les cellules sont des nucleus pulpopus, annulus fibrosis, ou un mélange de celles-ci.

15. Composition selon la revendication 12, dans laquelle les cellules sont des cellules souches embryonnaires non humaines ou des cellules souches dérivées d'un tissu choisi dans le groupe consistant en moelle épinière, tissu adipeux, muscle, cerveau, peau, foie, muscle lisse vasculaire, endothélium, sang ou placenta.

16. Composition selon la revendication 12, dans laquelle les cellules sont des cellules primaires, des cellules différenciées, des cellules génétiquement modifiées, des hybridomes, des cellules immortalisées, des cellules transformées, des cellules de fragment de tissu, des organites, ou un de leurs mélanges, des cellules nucléées, des cellules énucléées, des cellules de germe, des cellules de plaquette, des vésicules matricielles, des vésicules cellulaires, de la pâte d'os déminéralisée, des copeaux d'os, des fragments de cartilage ou des fragments de cellule ou fragments de tissu.

17. Composition selon la revendication 12, dans laquelle les cellules sont des cellules autologues, des cellules allogéniques ou des cellules xénogéniques.

18. Composition selon la revendication 12, dans laquelle le facteur biologiquement actif est un facteur d'attachement cellulaire choisi dans le groupe consistant en fibrinogène, fibrine, fibronectine, acide hyaluronique, héparine, collagène, polylysine, polyornithine, peptide cyclique de liaison à des récepteurs, protéine de liaison à des récepteurs.

19. Composition selon la revendication 12, dans laquelle le facteur biologiquement actif est une enzyme, un facteur de croissance ou une substance immobilisée par facteur de croissance.

20. Composition selon la revendication 12, dans laquelle le facteur biologiquement actif est un ADN plasmidique sous forme de liposomes, un complexe lipidique, un complexe de chitosan, un complexe de polylysine, un complexe DEAE dextrane.

21. Composition selon la revendication 12, dans laquelle le facteur biologiquement actif est un vaccin.

22. Composition selon la revendication 21, dans laquelle le vaccin comprend une particule virale infectieuse.

23. Composition selon la revendication 12, dans laquelle le facteur biologiquement actif est un facteur nutritif ou métabolique.

24. Composition selon la revendication 23, dans laquelle le facteur nutritif ou métabolique est un lipide, des acides aminés, et un cofacteur choisi dans le groupe consistant en cholestérol, glutamine, glucosamine, acide ascorbique, pyruvate et lactate.

25. Composition selon la revendication 12, dans laquelle le facteur biologiquement actif est au moins un élément choisi dans le groupe consistant en sang périphérique, sang osseux, sang du cordon, produit sanguin, cellules véhiculées par le sang, sérum, plaquettes, plasma riche en plaquette, fibrinogène, facteur de coagulation, et une enzyme véhiculée par le sang.

26. Composition selon la revendication 12, dans laquelle le facteur biologiquement actif est une substance ostéogénique.

27. Composition selon la revendication 26, dans laquelle la substance ostéogénique est un élément de la famille des protéines morphogénétiques osseuses choisi dans le groupe consistant en TGF-pl, BMP-2, BMP-6, BMP-7, ou l'un de leurs mélanges.

28. Composition selon la revendication 1, dans laquelle le polymère contenant de l'hydroxyle est le poly(alcool vinylique), le dextrane, lié à un aldéhyde réactif bifonctionnel.

29. Utilisation de la composition selon l'une quelconque des revendications 1 à 28, pour la préparation d'un médicament destiné à réparer du tissu mou.

30. Utilisation de la composition selon la revendication 12 ou 13, pour la préparation d'un médicament destiné à une administration spécifique au site dudit facteur biologiquement actif.

31. Utilisation de la composition selon l'une quelconque des revendications 1 à 28, pour la préparation d'un médicament destiné à la réparation osseuse.

32. Utilisation de la composition selon l'une quelconque des revendications 1 à 28, pour la préparation d'un médicament destiné à réparer ou resurfacer un cartilage endommagé.

33. Utilisation de la composition selon l'une quelconque des revendications 1 à 28, pour la préparation d'un médicament destiné à réparer un ménisque.

34. Composition selon la revendication 1, dans laquelle le milieu physiologique comprend des nutriments cellulaires choisis dans le groupe consistant en glucose, acides aminés, et vitamines, ou l'une de leurs combinaisons, à un pH isotonique et neutre.
